# EUROPÄISCHE PATENTSCHRIFT

(11) **EP 3 755 242 B1**
(45) Veröffentlichungstag und Bekanntmachung des Hinweises auf die Patenterteilung: **03.07.2024**
(21) Anmeldenummer: 19706969.3
(22) Anmeldetag: 21.02.2019
(51) Int. Cl.: A61B 17/122

(54) **CHIRURGISCHER CLIP**
SURGICAL CLIP
PINCE CHIRURGICALE

(30) Priorität: 21.02.2018 DE 102018103903
(43) Veröffentlichungstag der Anmeldung: 30.12.2020
(73) Patentinhaber: Aesculap AG, 78532 Tuttlingen (DE)
(72) Erfinder: SAUTER, Wolfgang, 78603 Renquishausen (DE); PLEIL, Thomas, 78073 Bad Dürrheim (DE)
(74) Vertreter: Hoeger, Stellrecht & Partner Patentanwälte mbB
(86) Internationale Anmeldenummer: PCT/EP2019/054274
(87) Internationale Veröffentlichungsnummer: WO 2019/162359

(56) Entgegenhaltungen:
- WO-A1-87/06118
- WO-A1-2007/006140
- WO-A1-2014/001008
- DE-A1- 3 139 488
- DE-U1-202006 010 414
- US-A- 3 827 438
- US-A- 4 796 625

## Beschreibung

Die vorliegende Erfindung betrifft einen chirurgischen Clip umfassend einen ersten Klemmarm, einen zweiten Klemmarm und ein vorspannendes Element, welcher erste Klemmarm eine erste Klemmfläche definiert, welcher zweite Klemmarm eine zweite Klemmfläche definiert und welches vorspannende Element die erste Klemmfläche und die zweite Klemmfläche in einer Grundstellung gegeneinander hält, insbesondere unter Vorspannung, wobei der erste und der zweite Klemmarm gegeneinander entgegen der Wirkung des vorspannenden Elements verschwenkbar sind, wobei das vorspannende Element in Form einer Schraubenfeder ausgebildet ist, welche eine Schraubenfederlängsachse definiert und ein erstes Schraubenfederende und ein zweites Schraubenfederende aufweist, wobei die Schraubenfeder zwischen dem ersten Schraubenfederende und dem zweiten Schraubenfederende mindestens eine sich über einen Umfangswinkel von mehr als 360° erstreckende Windung umfasst, wobei das erste Schraubenfederende über einen ersten Verbindungsabschnitt mit dem ersten Klemmarm verbunden ist, wobei das zweite Schraubenfederende über einen zweiten Verbindungsabschnitt mit dem zweiten Klemmarm verbunden ist, wobei die in der Grundstellung gegeneinander gehaltenen Klemmflächen eine Klemmebene definieren und wobei die Schraubenfederlängsachse parallel zur Klemmebene verläuft.

Chirurgische Clips der eingangs beschriebenen Art werden in der Chirurgie insbesondere zur Behandlung von Aneurysmen eingesetzt. Mit solchen sogenannten "Aneurysmen-Clips" werden beispielsweise Aneurysmen, also Aussackungen von Hohlorganen wie zum Beispiel Blutgefäßen abgeklemmt, indem die Aussackung zwischen den Klemmflächen der beiden Klemmarme abgeklemmt wird.

Bei bekannten chirurgischen Clips wird die Schraubenfeder zwischen den beiden Verbindungsabschnitten durch Wickeln ausgebildet. Je nachdem, unter welchem Winkel die beiden Verbindungsabschnitte von der Schraubenfeder abstehen, wird somit mindestens eine Windung ausgebildet, die sich über einen Umfangswinkel von mehr als 360° erstreckt, beispielsweise über einen Umfangswinkel von mehr als 500°.

Durch die herkömmliche Art der Wicklung der Schraubenfeder mit einer sich über einen Umfangswinkel von etwa 540° erstreckenden Windung ergibt sich das Problem, dass die an beiden freien Enden eines langgestreckten Rohlings ausgebildeten, parallel zueinander ausgerichteten Klemmflächen der beiden Klemmarme nach dem Wickeln der Schraubenfeder um etwa 15° gegeneinander geneigt sind. Dies kann insbesondere zu Problemen beim Öffnen und Schließen des Clips und dazu führen, dass die Klemmflächen der Klemmarme in der Grundstellung nicht perfekt aneinander anliegen. Außerdem kann der sogenannte "Scissoring-Effekt" auftreten kann, also das scherenähnliche aneinander Abgleiten der beiden Klemmarme, wodurch das abzuklemmende Hohlorgan verletzt werden kann.

Aus der US 3,827,438 ist ein Aneurysmen-Clip bekannt. Ein chirurgischer Clip ist in der DE 20 2006 010 414 U1 offenbart. In der WO 2007/006140 A1 ist ein bioaktiver Aneurysmen-Clip beschrieben. Ein vaskulärer Clip ist aus der WO 87/06118 A1 bekannt. In der WO 2014/001008 A1 ist ein chirurgischer Clip mit Innenfeder offenbart. Weitere Aneurysmen-Clips sind in der DE 31 39 488 A1 und in der US 4,796,625 beschrieben.

Es ist daher eine Aufgabe der vorliegenden Erfindung, einen chirurgischen Clip der eingangs beschriebenen Art besser handhabbar zu machen.

Diese Aufgabe wird bei einem chirurgischen Gerät der eingangs beschriebenen Art erfindungsgemäß dadurch gelöst, dass der erste Verbindungsabschnitt im Übergangsbereich zum ersten Schraubenfederende abgekröpft ist unter Ausbildung einer Abkröpfung und dass der zweite Verbindungsabschnitt im Übergangsbereich zum zweiten Schraubenfederende abgekröpft ist unter Ausbildung einer Abkröpfung, um die Schraubenfederlängsachse parallel zur Klemmfläche auszurichten, und dass die beiden Abkröpfungen als doppelte Abwinklungen des jeweiligen Verbindungsabschnitts zum jeweiligen Schraubenfederende ausgebildet sind.

Anders als bei am Markt verfügbaren, einstückig ausgebildeten chirurgischen Clips ist also die Schraubenfederlängsachse nicht relativ zur Klemmebene um wie oben beschrieben um etwa 15° geneigt, sondern verläuft nun genau parallel zur Klemmebene. Dadurch kann vermieden werden, dass, anders als bei den zueinander geneigten Klemmflächen herkömmlicher Clips, die Schraubenfeder nicht eine zur theoretisch idealen Arbeitsrichtung um 15° geneigte Kraftkomponente erfährt, welche dazu führen kann, dass die Schraubenfeder mit ihrer mindestens einen Windung "auf Block" gedrückt wird. Mit anderen Worten wird durch die besondere Ausrichtung der Schraubenfederlängsachse parallel zur Klemmebene gerade diese auf die mindestens eine Windung wirkende Kraftkomponente reduziert. Ferner kann so auch eine Ausdehnung der Schraubenfeder parallel zur Schraubenfederlängsachse, also parallel zur Klemmebene, minimiert werden. Durch die beschriebene Ausrichtung der Schraubenfederlängsachse wird eine effizientere Krafteinleitung in die Schraubenfeder erreicht. Zudem sorgen insbesondere geringere Torsionsspannungen in der Schraubenfeder für größere Sicherheit und einen größeren Arbeitsbereich derselben. Durch eine geringere Ausdehnung der Schraubenfeder parallel zur Schraubenfederlängsachse ergibt sich beim Applizieren des chirurgischen Clips eine bessere Sicht für einen Operateur. Außerdem lässt sich insbesondere der beschriebene "Scissoring-Effekt" reduzieren, da die Schraubenfeder stets so wirkt, dass die Klemmflächen der beiden Klemmarme senkrecht gegeneinander gedrückt werden. Gemäß der Erfindung ist der erste Verbindungsabschnitt im Übergangsbereich zum ersten Schraubenfederende abgekröpft und ist der zweite Verbindungsabschnitt im Übergangsbereich zum zweiten Schraubenfederende abgekröpft. Zwei solche Abkröpfungen in den beiden beschriebenen Übergangsbereichen, insbesondere auf ein Niveau, welches der Hälfte einer Erstreckung der Schraubenfeder parallel zur Schraubenfederlängsachse entspricht, ermöglicht auf einfache Weise eine Ausrichtung der Schraubenfederlängsachse parallel zur Klemmebene. Wie bereits oben beschrieben kann so eine die mindestens eine Windung der Schraubenfeder gegeneinander drückende Kraft und zudem auch eine Ausdehnung der Schraubenfeder in einer Richtung parallel zur Schraubenfederlängsachse minimiert werden. Unter Abkröpfen ist eine doppelte Abwinklung des jeweiligen Verbindungsabschnitts zum jeweiligen Schraubenfederende zu verstehen, um insbesondere die Schraubenfederlängsachse parallel zur Klemmfläche auszurichten.

Günstig ist es, wenn der erste Klemmarm ein erstes freies Ende aufweist, welches in einer Richtung vom vorspannenden Element weg oder im Wesentlichen vom vorspannenden Element weg weist. Diese Ausgestaltung ermöglicht es insbesondere, den chirurgischen Clip seitlich über ein abzuklemmendes Hohlorgan zu führen.

Vorteilhaft ist es, wenn der zweite Klemmarm ein zweites freies Ende aufweist, welches in einer Richtung vom vorspannenden Element weg oder im Wesentlichen vom vorspannenden Element weg weist. Dies ermöglicht es insbesondere, einen chirurgischen Clip auszubilden, welcher seitlich über ein Hohlorgan geführt werden kann, um dieses abzuklemmen.

Für eine einfache Handhabung des chirurgischen Clips ist es günstig, wenn der Clip einen Kreuzungsbereich umfasst und wenn sich der erste Verbindungsabschnitt und der zweite Verbindungsabschnitt im Kreuzungsbereich kreuzen. Insbesondere ermöglicht es eine solche Ausgestaltung, den Clip durch Bewegen der beiden Verbindungsabschnitte aufeinander zu zu öffnen. Auf diese Weise kann der Clip durch einen Operateur einfach und sicher gehandhabt werden.

Gemäß einer weiteren bevorzugten Ausführungsform der Erfindung kann vorgesehen sein, dass der erste Verbindungsabschnitt und/oder der zweite Verbindungsabschnitt eine auf den anderen Verbindungsabschnitt hin weisende Abflachung aufweisen. Insbesondere können zwei Abflachungen vorgesehen sein, die aneinander anliegen oder durch einen schmalen Spalt voneinander beabstandet sind. Auf diese Weise lässt sich ein besonders kompakter chirurgischer Clip ausbilden.

Vorteilhaft ist es, wenn die Abflachung des ersten und/oder zweiten Verbindungsabschnitts eine Abflachungsebene definiert und wenn die Abflachungsebene senkrecht oder im Wesentlichen senkrecht zur Klemmebene verläuft. Auf diese Weise kann der chirurgische Clip insbesondere sicher geöffnet und geschlossen werden, ohne dass es zwingend zu Berührungen der beiden Verbindungsabschnitte kommen muss. Die Abflachungen können insbesondere auch als gegenseitige Führung der Verbindungsabschnitte beim Öffnen und Schließen des chirurgischen Clips dienen.

Günstig ist es, wenn sich die Abflachung des ersten Verbindungsabschnitts direkt an den ersten Klemmarm anschließt und/oder wenn sich die Abflachung des zweiten Verbindungsabschnitts direkt an den zweiten Klemmarm anschließt. Auf diese Weise können die Klemmarme im Wesentlichen entlang ihrer gesamten Längserstreckung mit den beiden Klemmflächen in der Grundstellung aneinander anliegen.

Günstig ist es, wenn der erste Verbindungsabschnitt zwischen dem ersten Klemmarm und dem ersten Schraubenfederende um einen ersten Knickwinkel abgewinkelt ist und/oder wenn der zweite Verbindungsabschnitt zwischen dem zweiten Klemmarm und dem zweiten Schraubenfederende um einen zweiten Knickwinkel abgewinkelt ist. Diese Ausgestaltung ermöglicht es insbesondere, das erste Schraubenfederende und das zweite Schraubenfederende um einen Umfangswinkel von etwa 180° versetzt zueinander auszubilden und mit dem ersten beziehungsweise zweiten Verbindungsabschnitt zu verbinden.

Um einen insbesondere für eine Handhabung symmetrischen chirurgischen Clip auszubilden zu können, ist es vorteilhaft, wenn der erste Knickwinkel dem zweiten Knickwinkel entspricht oder im Wesentlichen entspricht. So kann ein Operateur den chirurgischen Clip intuitiv handhaben, auch wenn er um eine von den Klemmarmen definierte Längsachse um 180° verdreht in seiner Hand liegt.

Günstigerweise definieren der erste und/oder der zweite Knickwinkel einen Innenwinkel in einem Bereich von etwa 90° bis etwa 120°. Auf diese Weise kann ein besonders kompakter chirurgischer Clip ausgebildet werden.

Gemäß einer weiteren bevorzugten Ausführungsform der Erfindung kann vorgesehen sein, dass der erste Verbindungsabschnitt im Übergangsbereich zum ersten Klemmarm um einen dritten Knickwinkel abgewinkelt ist und/oder wenn der zweite Verbindungsabschnitt im Übergangsbereich zum zweiten Klemmarm um einen vierten Knickwinkel abgewinkelt ist. Auf diese Weise ist es insbesondere möglich, den Verbindungsabschnitt so auszubilden, dass er voneinander beabstandete Teilabschnitte aufweist, wenn die Klemmflächen in der Grundstellung aneinander anliegen. Diese Teilabschnitte können dann gegeneinander bewegt werden, um den Clip zu öffnen. Gleichzeitig können diese Teilabschnitte insbesondere auch gegenseitig Anschläge bilden, um das Öffnen des chirurgischen Clips zu begrenzen.

Günstigerweise entspricht oder im Wesentlichen entspricht der dritte Knickwinkel dem vierten Knickwinkel. So lässt sich insbesondere ein symmetrischer Clip ausbilden. Ferner kann insbesondere auch vorgesehen sein, dass der dritte Knickwinkel und/oder der vierte Knickwinkel dem ersten und/oder zweiten Knickwinkel entsprechen. Insbesondere können die ersten und zweiten Knickwinkel einerseits und die dritten und vierten Knickwinkel andererseits Wechselwinkel im mathematischen Sinn definieren.

Gemäß einer weiteren bevorzugten Ausführungsform der Erfindung kann vorgesehen sein, insbesondere auch bei einem chirurgischen Clip der eingangs beschriebenen Art, dass die mindestens eine Windung mindestens einen ersten Windungsabschnitt und mindestens einen zweiten Windungsabschnitt umfasst und dass der mindestens eine erste Windungsabschnitt eine erste Windungsabschnittsebene definiert und/oder dass der mindestens eine zweite Windungsabschnitt eine zweite Windungsebene definiert und dass der mindestens eine erste Windungsabschnitt und der mindestens eine zweite Windungsabschnitt über einen vom ersten Schraubenfederende und vom zweiten Schraubenfederende entfernten Kröpfungsabschnitt miteinander verbunden sind. Die beschriebene Ausgestaltung ermöglicht es insbesondere, durch das Ausbilden des Kröpfungsabschnitts die beiden Windungsabschnitte so voneinander zu beabstanden, dass sich diese nicht berühren. Dies wird insbesondere dadurch erreicht, dass der Kröpfungsabschnitt, der den mindestens einen ersten Windungsabschnitt und den mindestens einen zwischen Windungsabschnitt miteinander verbindet, zwischen diesen angeordnet ist und auf diese Weise eine direkte Verbindung des mindestens einen ersten Windungsabschnitts und des mindestens einen zweiten Windungsabschnitts ausbildet. Mit anderen Worten ist der Kröpfungsabschnitt in die mindestens eine Windung integriert. Insbesondere bei einem Schließen der Schraubenfeder, bei dem sich ein Durchmesser derselben verringert und die mindestens eine Windung in einer Richtung parallel zur Schraubenachse aufeinander zu bewegt wird, kann so ein Blockieren der Schraubenfeder durch das "auf Block" Drücken verhindert werden, da benachbarte Windungsabschnitte der mindestens einen Windung nicht mehr miteinander in Kontakt kommen können, insbesondere beim Öffnen des chirurgischen Clips. Auf diese Weise lässt sich insbesondere auch eine Reibung im Bereich der Schraubenfeder minimieren, was insbesondere das Öffnen des chirurgischen Clips, wenn dieser sich kreuzende Verbindungsabschnitte aufweist, vereinfacht. Außerdem ermöglicht es die Ausgestaltung der ersten und zweiten Windungsabschnitte derart, dass sie jeweils eine Windungsabschnittsebene definieren, dass sich bei einer einwirkenden Kraft zum Öffnen des chirurgischen Clips die Schraubenfeder nicht weiter tordieren kann, sondern ein Zusammenziehen der Schraubenfeder im Wesentlichen jeweils nur im Bereich der ebenen Windungsabschnitte erfolgen kann. Durch eine Länge und eine Form des Kröpfungsabschnitts kann insbesondere ein gewünschter Abstand zwischen dem mindestens einen ersten Windungsabschnitt und dem mindestens einen zweiten Windungsabschnitt eingestellt werden. Insbesondere kann der Kröpfungsabschnitt ein Abstandshalteelement bilden, um den mindestens einen ersten Windungsabschnitt und den mindestens einen zweiten Windungsabschnitt voneinander beabstandet zu halten, so dass sich diese nicht berühren, und zwar insbesondere unabhängig davon, ob der Clip geschlossen oder geöffnet ist. Selbstverständlich kann der chirurgische Clip auch eine Schraubenfeder mit mehr als zwei Windungsabschnitten aufweisen. Vorzugsweise sind dann jeweils zwei benachbarte Windungsabschnitte über einen diese verbindenden Kröpfungsabschnitt miteinander verbunden.

Besonders einfach ausbilden lässt sich der chirurgische Clip, wenn der Kröpfungsabschnitt geradlinig oder im Wesentlichen geradlinig ausgebildet ist und an einem ersten Kröpfungsabschnittsende abgewinkelt mit dem mindestens einen ersten Windungsabschnitt verbunden ist und an einem zweiten Kröpfungsabschnittsende abgewinkelt mit dem mindestens einen zweiten Windungsabschnitt verbunden ist. Vorzugsweise ist die Abwinklung zwischen dem Kröpfungsabschnittsende und dem jeweiligen Windungsabschnitt identisch.

Vorzugsweise verläuft der Kröpfungsabschnitt quer zur ersten und/oder zweiten Windungsabschnittsebene. Insbesondere kann er unter einem Kröpfungsabschnittswinkel von etwa 45° verlaufen. Dies ermöglicht es insbesondere, die Schraubenfeder ohne zu große plastische Verformung in der gewünschten Weise auszubilden, nämlich mit voneinander beabstandeten Windungsabschnitten.

Um die Schraubenfederlängsachse auf einfache definierte Weise parallel zur Klemmebene auszurichten, ist es günstig, wenn die erste Windungsabschnittsebene und/oder die zweite Windungsabschnittsebene senkrecht zur Schraubenfederlängsachse verlaufen.

Vorteilhaft ist es, wenn sich der mindestens eine erste Windungsabschnitt über einen ersten Windungsabschnittsumfangswinkel von weniger als 360°, insbesondere weniger als 300°, erstreckt und/oder wenn sich der mindestens eine zweite Windungsabschnitt über einen zweiten Windungsabschnittsumfangswinkel von weniger als 360°, insbesondere weniger als 300°, erstreckt. Auf diese Weise lässt sich eine Schraubenfeder insbesondere mit mindestens zwei derartigen Windungsabschnitten ausbilden, die parallel zueinander ausgerichtet sind und Windungsabschnittsebenen definieren, die senkrecht zur Schraubenfederlängsachse verlaufen. Insbesondere kann die Schraubenfeder auch drei, vier oder mehr derartige Windungsabschnitte umfassen.

Vorteilhaft ist es, wenn der mindestens eine erste Windungsabschnitt und der mindestens eine zweite Windungsabschnitt durch einen Windungsabschnittsspalt voneinander getrennt sind. Wie bereits oben beschrieben hat diese Ausgestaltung den Vorteil, dass sich bei einem Öffnen des Clips, welcher sich kreuzende Verbindungsabschnitte aufweist, die Schraubenfeder im Durchmesser verkleinert, benachbarte Windungsabschnitte nicht miteinander in Kontakt kommen können. Dadurch kann eine Reibung im Bereich zwischen den Windungsabschnitten der Schraubenfeder minimiert oder sogar ganz vermieden werden. So kann eine von der Schraubenfeder ausübbare vorspannende Kraft bei der Herstellung definiert vorgegeben werden und kann beim Applizieren durch einen Operateur nicht versehentlich geändert werden. Der Windungsabschnittsspalt kann insbesondere eine Breite in einem Bereich zwischen 0 mm und etwa 1 mm aufweisen.

Günstig ist es, wenn die Schraubenfeder einen Schraubenfederdurchmesser definiert, wenn der erste Klemmarm und der zweite Klemmarm eine Klemmarmlänge aufweisen und wenn der Schraubenfederdurchmesser kleiner als die Klemmarmlänge ist. Unter dem Schraubenfederdurchmesser ist insbesondere ein Durchmesser zu verstehen, welcher durch die mindestens eine Windung der Schraubenfeder definiert wird, also ein Durchmesser in einer Ebene, welche senkrecht zur Klemmebene und senkrecht zur Schraubenfederlängsachse verläuft.

Um ein Hohlorgan sicher zwischen den beiden Klemmarmen klemmen zu können, ist es vorteilhaft, wenn die erste Klemmfläche und/oder die zweite Klemmfläche eine Klemmflächenstruktur aufweisen. Insbesondere kann die Klemmflächenstruktur makroskopisch und/oder mikroskopisch ausgebildet sein. Auf diese Weise kann insbesondere ein abrutschen des chirurgischen Clips vom abzuklemmenden Hohlorgan vermieden werden.

Auf einfache Weise ausbilden lässt sich der chirurgische Clip, wenn die Klemmflächenstruktur Klemmvorsprünge und/oder Klemmausnehmungen umfasst. Insbesondere können die Klemmvorsprünge und/oder die Klemmausnehmungen linien- und/oder punktförmig angeordnet oder ausgebildet sein.

Einfach und kostengünstig ausbilden lässt sich der chirurgische Clip, wenn er einstückig, insbesondere monolithisch, ausgebildet ist. Beispielsweise kann er monolithisch aus einem einzigen Rohling ausgebildet werden.

Günstig ist es, wenn der Clip aus einem Federstahldraht durch Umformen ausgebildet ist. Insbesondere kann der Federstahldraht einen kreisförmigen oder im Wesentlichen kreisförmigen Querschnitt aufweisen. Das Umformen kann insbesondere durch Pressformen erfolgen. So kann der chirurgische Clip insbesondere aus einem Rohling aus einem Federstahldraht ausgebildet werden. Insbesondere kann der chirurgische Clip vollständig manuell oder vollständig maschinell hergestellt werden.

Gemäß einer weiteren bevorzugten Ausführungsform der Erfindung kann vorgesehen sein, dass eine Querschnittsfläche des Clips im Bereich der Schraubenfeder kleiner ist als im Bereich des ersten und/oder zweiten Verbindungsabschnitts und/oder im Bereich des ersten und/oder des zweiten Klemmarms. So lässt sich insbesondere eine besonders kompakte Schraubenfeder ausbilden, die beispielsweise auch eine verbesserte Sicht bei einer Applikation des chirurgischen Clips an ein Hohlorgan ermöglicht.

Die nachfolgende Beschreibung bevorzugter Ausführungsformen der Erfindung dient im Zusammenhang mit der Zeichnung der näheren Erläuterung. Es zeigen:
- Figur 1a:: eine perspektivische Ansicht eines chirurgischen Clips, wie er aus dem Stand der Technik bekannt ist, vor dem Überkreuzen der beiden Verbindungsabschnitte beziehungsweise der beiden Klemmarme;
- Figur 1b:: eine Ansicht der Anordnung aus Figur 1a in Richtung des Pfeils A;
- Figur 2:: eine perspektivische Ansicht eines ersten Ausführungsbeispiels eines verbesserten chirurgischen Clips;
- Figur 3:: eine Ansicht des Clips aus Figur 2 in Richtung des Pfeils B;
- Figur 4:: eine Ansicht des Clips aus Figur 3 in Richtung des Pfeils C;
- Figur 5:: eine Ansicht des Clips aus Figur 4 in Richtung des Pfeils D;
- Figur 6:: eine Schnittansicht längs Linie 6-6 in Figur 4;
- Figur 7:: eine Schnittansicht längs Linie 7-7 in Figur 4;
- Figur 8:: eine perspektivische Ansicht eines zweiten Ausführungsbeispiels eines verbesserten chirurgischen Clips;
- Figur 9:: eine Ansicht des Clips aus Figur 8 in Richtung des Pfeils E;
- Figur 10:: eine Ansicht des Clips aus Figur 9 in Richtung des Pfeils F;
- Figur 11:: eine Ansicht des Clips aus Figur 10 in Richtung des Pfeils G;
- Figur 12:: eine Schnittansicht längs Linie 12-12 in Figur 10;
- Figur 13:: eine Schnittansicht längs Linie 13-13 in Figur 10;
- Figur 14:: eine perspektivische Ansicht eines dritten Ausführungsbeispiels eines verbesserten chirurgischen Clips;
- Figur 15:: eine Ansicht des Clips aus Figur 14 in Richtung des Pfeils H;
- Figur 16:: eine Ansicht des Clips aus Figur 15 in Richtung des Pfeils I;
- Figur 17:: eine Ansicht des Clips aus Figur 16 in Richtung des Pfeils K;
- Figur 18:: eine Schnittansicht längs Linie 18-18 in Figur 16; und
- Figur 19:: eine Schnittansicht längs Linie 19-19 in Figur 16.

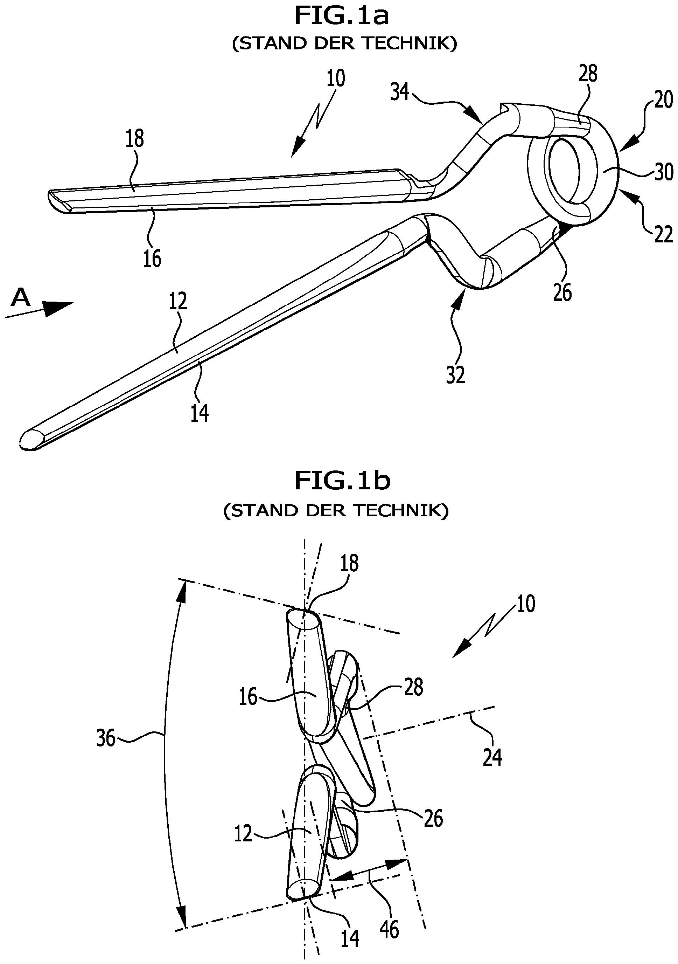

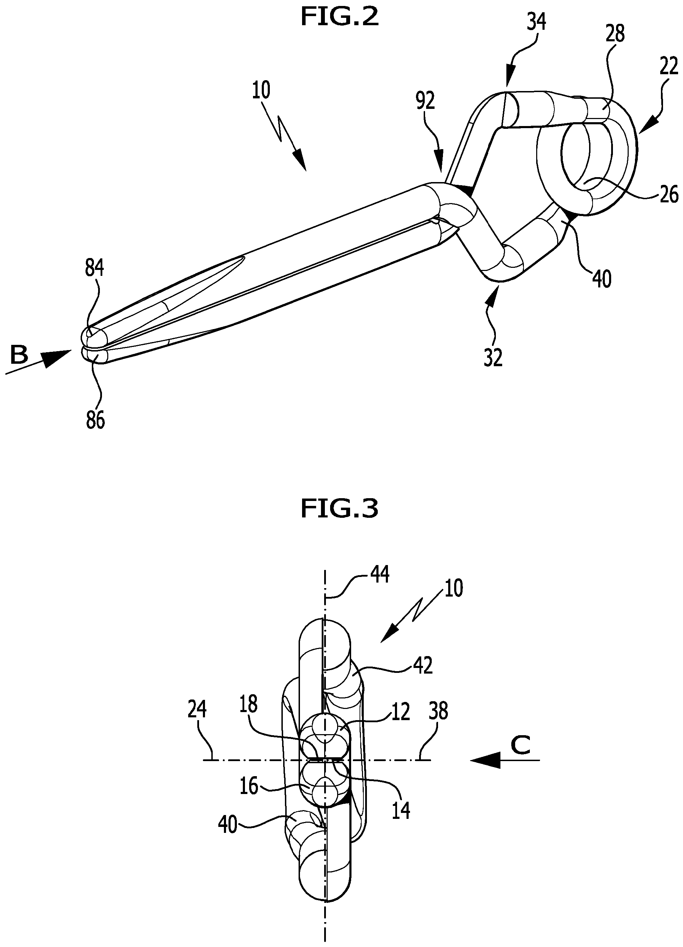

Die Figuren 1 und 2 zeigen beispielhaft einen insgesamt mit dem Bezugszeichen 10 bezeichneten chirurgischen Clip, wie er aus dem Stand der Technik bekannt ist. Er umfasst einen ersten Klemmarm 12 mit einer ersten Klemmfläche 14 und einen zweiten Klemmarm 16 mit einer zweiten Klemmfläche 18. Des Weiteren umfasst der Clip 10 ein vorspannendes Element 20, welches in Form einer Schraubenfeder 22 ausgebildet ist.

Das vorspannende Element 20 dient dazu, die erste Klemmfläche 14 und die zweite Klemmfläche 18 in einer Grundstellung gegeneinander zu halten, insbesondere unter Vorspannung.

In den Figuren 1a und 1b ist der Clip 10 jedoch nicht in der Grundstellung dargestellt, sondern mit nicht aneinander anliegenden Klemmflächen 14 und 18 direkt nach der Herstellung des Clips 10. Die Klemmarme 12 und 16 sind aufgrund der Wirkung der Schraubenfeder 22 etwas voneinander weg gespreizt. Nach dem Überkreuzen der Klemmarme 12 und 16 drückt dann die Schraubenfeder 22 die Klemmflächen 14 und 18 unter Vorspannung gegeneinander.

Die Schraubenfeder 22 definiert eine Schraubenfederlängsachse 24.

Die Schraubenfeder 22 weist ein erstes Schraubenfederende 26 und ein zweites Schraubenfederende 28 auf.

Zwischen dem ersten Schraubenfederende 26 und dem zweiten Schraubenfederende 28 umfasst die Schraubenfeder 22 mindestens eine Windung 30, die sich über einen Umfangswinkel von mehr als 360° erstreckt, bei dem in den Figuren 1a und 1b dargestellten Ausführungsbeispiel über einen Umfangswinkel von etwa 520°.

Das erste Schraubenfederende 26 ist über einen ersten Verbindungsabschnitt 32 mit dem ersten Klemmarm 12 verbunden. Das zweite Schraubenfederende 28 ist über einen zweiten Verbindungsabschnitt 34 mit dem zweiten Klemmarm 16 verbunden.

Werden die Verbindungsabschnitte 32 und 34 aufeinander zu bewegt, so dass der zweite Klemmarm 16 den ersten Klemmarm 12 untergreifen kann, nimmt der Clip 10 die Grundstellung ein. Die Klemmflächen 14 und 18 liegen dann im Wesentlichen aneinander an und werden durch die Schraubenfeder 22 unter Vorspannung gegeneinander gedrückt gehalten.

Figur 1b zeigt ein wesentliches Problem bei chirurgischen Clips, wie sie aus dem Stand der Technik bekannt sind. Wird der Clip 10 aus einem Rohling aus Federstahldraht geformt, und zwar derart, dass beim Rohling die Klemmarme 12 und 16 mit parallel zueinander verlaufenden Klemmflächen 14 und 18 an freien Enden des Drahtrohlings ausgebildet werden, führt das Wickeln der Schraubenfeder 22 dazu, dass die Klemmflächen 14 und 18 um einen Winkel 36 gegeneinander geneigt sind. Dies hat zur Folge, dass die Klemmflächen 14 und 18 in der Grundstellung gerade nicht flächig aneinander anliegen, sondern im Wesentlichen nur linienförmig. Damit wirkt eine entsprechende Kraftkomponente unter dem Winkel 36 von etwa 15°, die aneinander anliegende Bereiche der Schraubenfeder 22 gegeneinander drückt.

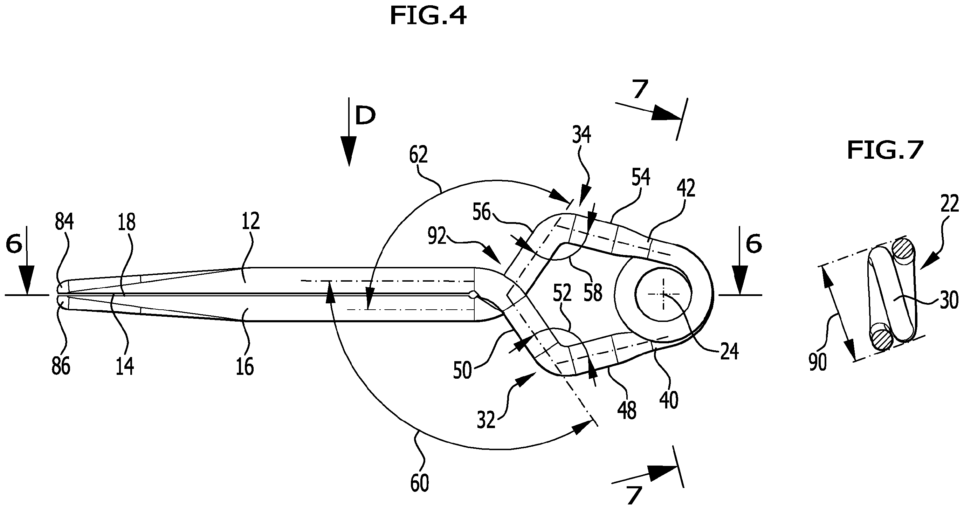

In den Figuren 2 bis 7 ist nun ein erstes Ausführungsbeispiel eines verbesserten chirurgischen Clips dargestellt, das ebenfalls mit dem Bezugszeichen 10 bezeichnet ist. Identische Teile des in den Figuren 2 bis 7 dargestellten Clips, die auch bereits bei dem aus der Stand den Technik bekannten und den Figu-ren 1a und 1b dargestellten Clip 10 vorhanden sind, sind der besseren Übersichtlichkeit wegen mit denselben Bezugszeichen bezeichnet.

Der Clip 10, wie in den Figuren 2 bis 7 dargestellt ist, unterscheidet sich vom Clip der Figuren 1a und 1b insbesondere dadurch, dass die Klemmflächen 14 und 18 in der in Figur 2 dargestellten Grundstellung flächig aneinander anliegen und eine Klemmebene 38 definieren.

Die Schraubenfederlängsachse 24 verläuft, anders als bei dem aus dem Stand der Technik bekannten Clip 10, parallel zur Klemmebene 38.

Um dies zu erreichen, ist das erste Schraubenfederende 26 im Übergangsbereich zum ersten Verbindungsabschnitt 32 abgekröpft. Mit anderen Worten ist eine erste Abkröpfung 40 ausgebildet, um den ersten Verbindungsabschnitt zur Schraubenfeder 22 auszurichten. In analoger Weise ist ein Übergangsbereich zwischen dem zweiten Schraubenfederende 28 und dem zweiten Verbindungsabschnitt 34 abgekröpft, so dass eine zweite Abkröpfung 42 ausgebildet ist.

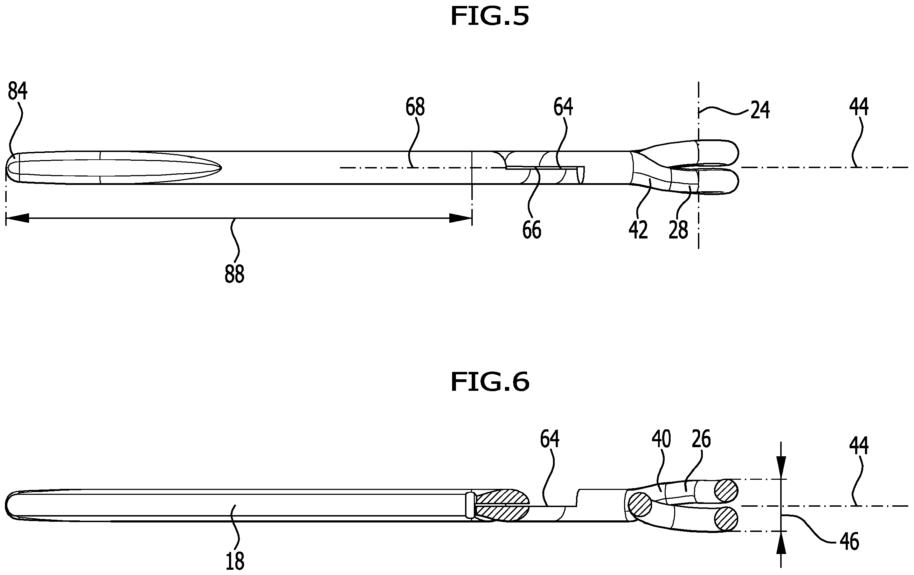

In den Figuren 5 und 6 ist gut zu erkennen, dass die beiden Klemmarme 12 und 16 durch die beiden Abkröpfungen 40 und 42 symmetrisch bezogen auf eine von der Schraubenfeder 22 definierte Schraubenfederebene 44 angeordnet sind. Eine maximale Dicke 46 der Schraubenfeder 22 parallel zur Schraubenfederlängsachse 24 ist daher kleiner als bei der aus dem Stand der Technik bekannten Schraubenfeder 10, die in den Figuren 1a und 1b dargestellt ist.

Der erste Verbindungsabschnitt 32 umfasst zwei geradlinige Abschnitte 48 und 50, die relativ zueinander um einen ersten Knickwinkel 52 abgewinkelt sind. Ebenso umfasst der zweite Verbindungsabschnitt 34 zwei geradlinige Abschnitte 54 und 56, die relativ zueinander um einen zweiten Knickwinkel 58 abgewinkelt sind.

Der Abschnitt 48 schließt sich direkt an die erste Abkröpfung 40 an, der Abschnitt 54 an die zweite Abkröpfung 42. Der Abschnitt 50 ist relativ zum ersten Klemmarm 12 um einen dritten Knickwinkel 60 abgewinkelt. In analoger Weise ist der Abschnitt 56 relativ zum zweiten Klemmarm 16 um einen vierten Knickwinkel 62 abgewinkelt.

Der erste Verbindungsabschnitt 32 ist ausgehend vom ersten Klemmarm 12 bis an den Abschnitt 48 heran mit einer Abflachung 64 versehen. In analoger Weise ist der zweite Verbindungsabschnitt 34 ausgehend vom zweiten Klemmarm 16 bis an den Abschnitt 54 heran mit einer weiteren Abflachung 66 versehen.

Die Abflachungen 64 und 66 weisen aufeinander zu und definieren eine gemeinsame Abflachungsebene 68, die sowohl senkrecht zur Schraubenfederlängsachse 24 als auch zur Klemmebene 38 verläuft. Die Abflachungsebene 68 und die Schraubenfederebene 44 fallen zusammen.

Die Verbindungsabschnitte 32 und 34 kreuzen sich im Bereich der Abflachungen 64 und 66, wodurch ein Kreuzungsbereich 92 definiert wird.

Die Abkröpfungen 40 und 42 dienen dazu, die beim Clip 10 gemäß dem Stand der Technik, wie er beispielhaft in den Figuren 1a und 1b dargestellt ist, nicht parallel zueinander verlaufenden Klemmflächen 14 und 18 parallel zueinander auszurichten, um weiter oben beschriebenen Vorteile durch diese Ausgestaltung zu erreichen.

Die Schraubenfeder 22 ist bei dem in den Figuren 2 bis 7 dargestellten Clip 10 identisch ausgebildet wie bei dem Clip 10 in den Figuren 1a und 1b.

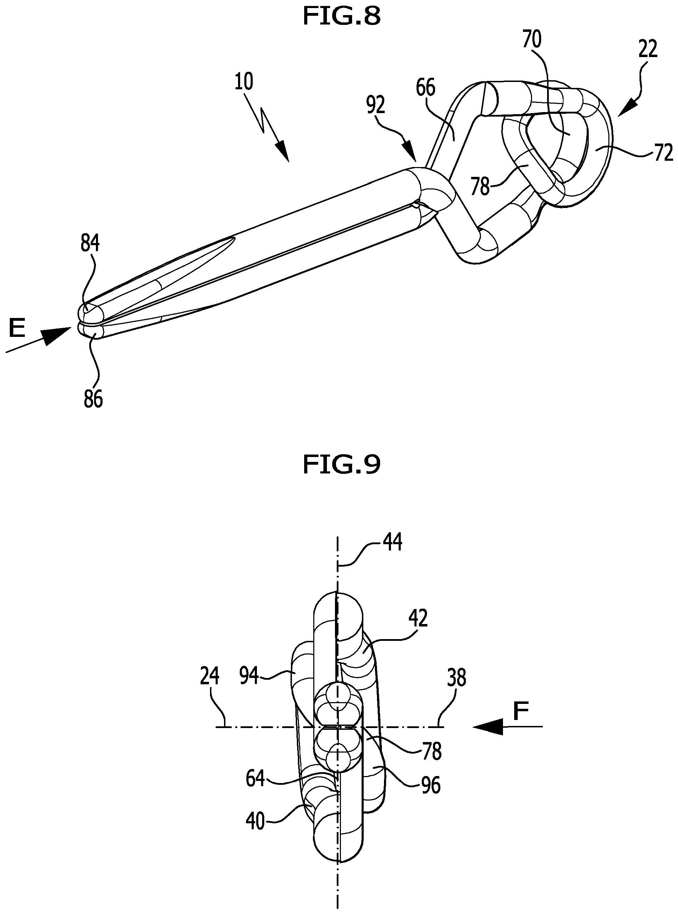

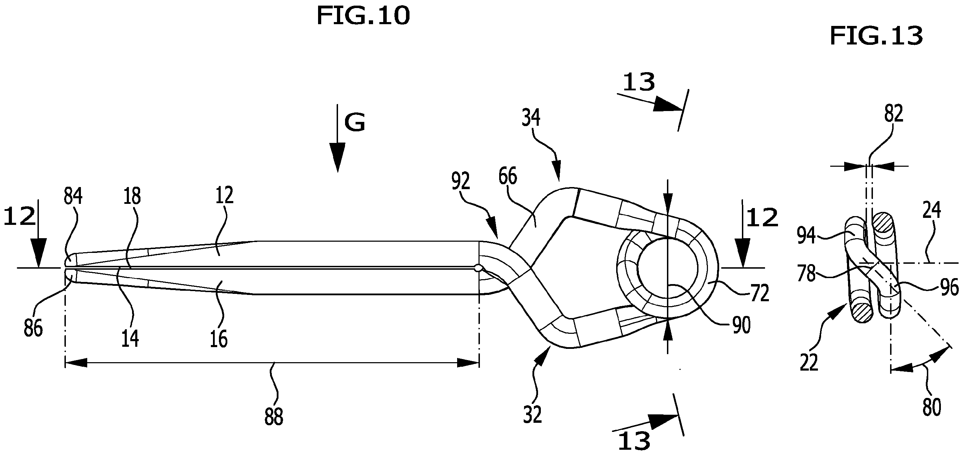

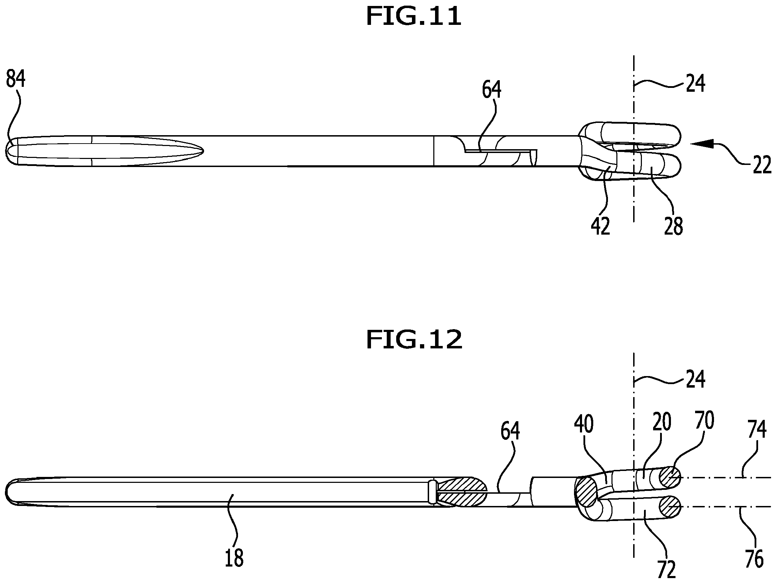

In den Figuren 8 bis 13 ist beispielhaft ein zweites Ausführungsbeispiel eines insgesamt mit dem Bezugszeichen 10 bezeichneten, verbesserten chirurgischen Clips dargestellt. Er unterscheidet sich von dem in den Figuren 2 bis 7 dargestellten Clip 10 durch die Ausgestaltung der Schraubenfeder 22.

Die Schraubenfeder 22 umfasst bei dem in den Figuren 8 bis 13 dargestellten Ausführungsbeispiel des Clips 10 einen ersten Windungsabschnitt 70 und einen zweiten Windungsabschnitt 72. Der erste Windungsabschnitt 70 definiert eine erste Windungsabschnittsebene 74, der zweite Windungsabschnitt 72 eine zweite Windungsabschnittsebene 76.

Der erste Windungsabschnitt 70 schließt sich an die erste Abkröpfung 40 an, der zweite Windungsabschnitt 72 an die zweite Abkröpfung 42.

Ferner sind der erste Windungsabschnitt 70 und der zweite Windungsabschnitt 72 direkt miteinander über einen Kröpfungsabschnitt 78 miteinander verbunden. Der Kröpfungsabschnitt 78 ist also direkt zwischen dem ersten Windungsabschnitt 70 und dem zweiten Windungsabschnitt 72 angeordnet beziehungsweise ausgebildet. Der Kröpfungsabschnitt 78 ist im Wesentlichen geradlinig ausgebildet und verläuft quer zu den beiden Windungsabschnittsebenen 74 und 76, und zwar bei dem in den Figuren 8 bis 13 dargestellten Ausführungsbeispiel des Clips 10 unter einem Kröpfungsabschnittswinkel 80 von etwa 45°.

Der Kröpfungsabschnitt 78 ist an einem ersten Kröpfungsabschnittsende 94 abgewinkelt und mit dem ersten Windungsabschnitt 70 verbunden. Ferner ist der Kröpfungsabschnitt an einem zweiten Kröpfungsabschnittsende abgewinkelt und mit dem zweiten Windungsabschnitt 72 verbunden.

Die Windungsabschnittsebenen 74 und 76 verlaufen parallel zueinander und senkrecht zur Schraubenfederlängsachse 24.

Wie insbesondere in Figur 8 gut zu erkennen, erstrecken sich die Windungsabschnitte 70 und 72 jeweils über einen Umfangswinkel von weniger als 360°, insbesondere weniger als 300°. Er beträgt vielmehr etwa nur 200°.

Durch den Kröpfungsabschnitt 78, der eine entsprechende Länge aufweist, sind die beiden Windungsabschnitte 70 und 72 voneinander beabstandet und durch einen Windungsabschnittsspalt 82 voneinander getrennt.

Werden die Verbindungsabschnitte 32 und 34 aufeinander zu bewegt, wird die Schraubenfeder 22 zusammengezogen. Durch den Kröpfungsabschnitt 78 wird jedoch sichergestellt, dass sich die beiden Windungsabschnitte 70 und 72, und zwar unabhängig von einer Öffnungsstellung des Clips 10, in welcher die Klemmarme 12 und 16 voneinander weg verschwenkt sind, einander nicht berühren können. Dadurch kann eine Reibung im Bereich der Schraubenfeder 22 minimiert oder sogar ganz ausgeschaltet werden.

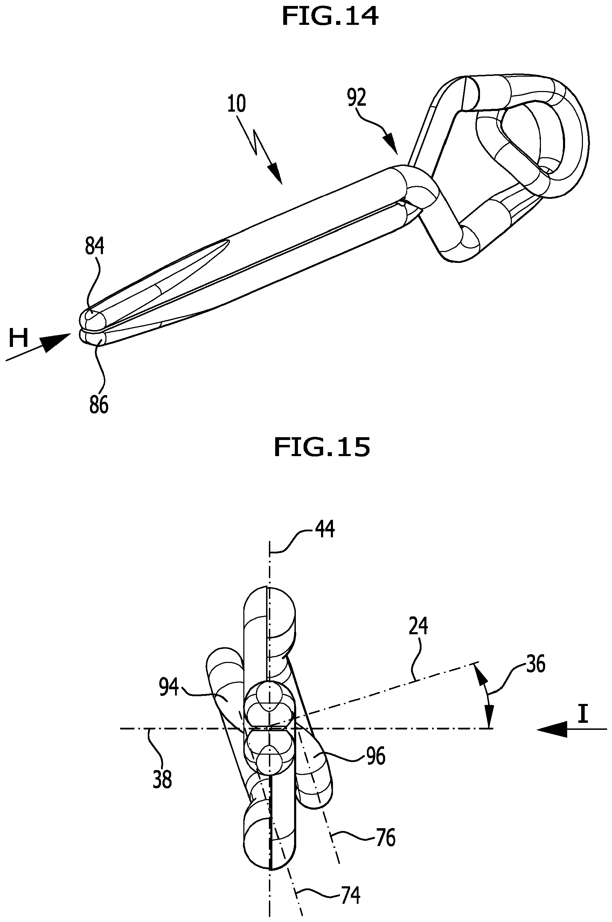

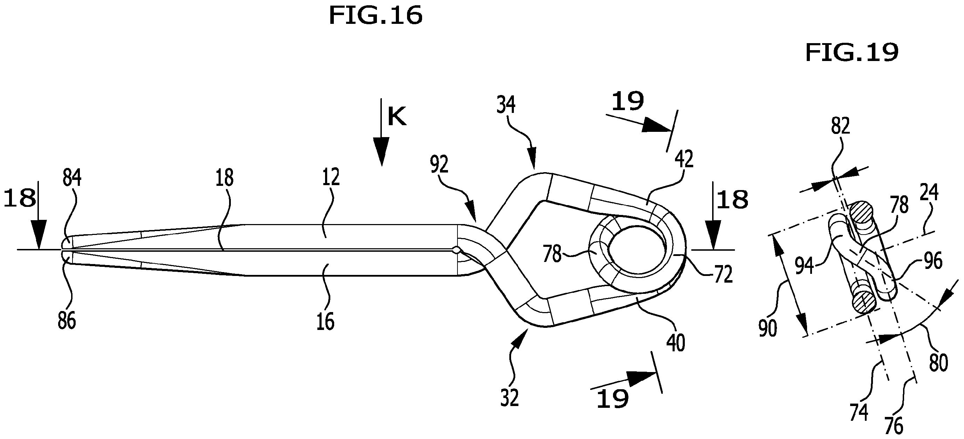

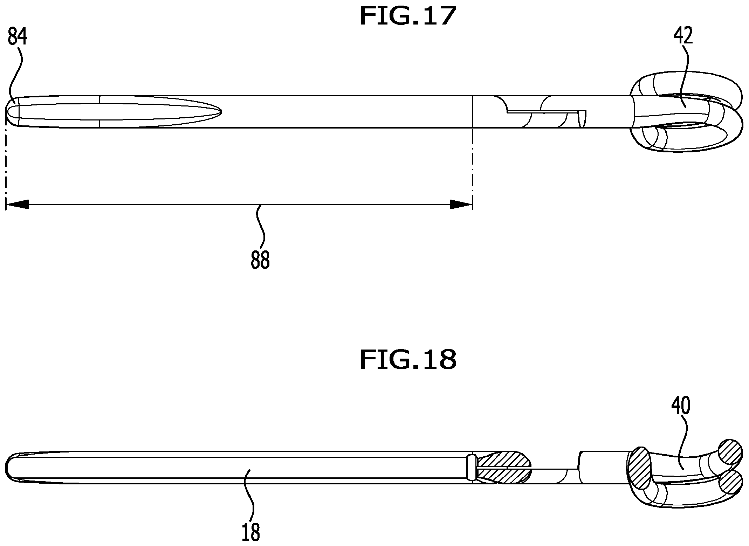

Ein drittes Ausführungsbeispiel eines verbesserten chirurgischen Clips 10 ist schematisch in den Figuren 14 bis 19 dargestellt. Es stimmt in seinem Aufbau im Wesentlichen mit dem zweiten Ausführungsbeispiel des verbesserten Clips 10, wie er in den Figuren 8 bis 13 dargestellt ist, überein.

Der wesentliche Unterschied zwischen dem Clip 10 gemäß den Figuren 14 bis 19 gegenüber dem Clip gemäß den Figuren 8 bis 13 liegt darin, dass die Windungsabschnitte 70 und 72 mit ihren Windungsabschnittsebenen 74 und 76 gegenüber der Schraubenfederebene 44 um den Winkel 36 geneigt sind. Mithin verläuft die Schraubenfederlängsachse 24 also nicht parallel zur Klemmebene 38, sondern ist gegenüber dieser um den Winkel 36 geneigt.

Somit vereint der Clip 10, wie er in den Figuren 8 bis 13 dargestellt ist, die besonderen Eigenschaften der Schraubenfeder 22 des Clips 10, wie er in den Figuren 2 bis 7 dargestellt ist, mit den besonderen Eigenschaften der Schraubenfeder 22 des Clips 10, wie er in den Figuren 14 bis 19 dargestellt ist.

Allen in den Figuren dargestellten Ausführungsbeispielen von Clips 10 ist gemein, dass der erste Klemmarm 12 ein erstes freies Ende 84 aufweist und der zweite Klemmarm 16 ein zweites freies Ende 86.

Optional können die erste Klemmfläche 14 und/oder die zweite Klemmfläche 18 eine in den Figuren nicht dargestellte Klemmflächenstruktur aufweisen. Diese kann insbesondere makroskopisch oder mikroskopisch ausgebildet sein.

Die Klemmflächenstruktur kann insbesondere Klemmvorsprünge und/oder Klemmausnehmungen umfassen. Diese können insbesondere linien- und/oder punktförmig ausgebildet sein.

Alle in den Figuren 2 bis 19 dargestellten Ausführungsbeispiele chirurgischer Clips 10 ermöglichen eine verbesserte Handhabung gegenüber aus dem Stand der Technik bekannter chirurgischer Clips 10. Dies liegt zum einen an der optional parallel zur Klemmebene 38 ausgerichteten Schraubenfederlängsachse 24 beziehungsweise am optional vorgesehenen Kröpfungsabschnitt 78, der die Windungsabschnitte 70 und 72 direkt miteinander verbindet und derart voneinander beabstandet hält, dass der Windungsabschnittsspalt 82 ausgebildet wird.

Alle verbesserten Clips 10 sind einstückig, nämlich monolithisch, ausgebildet.

Alle verbesserten Clips 10 sind aus einem Rohling aus einem Federstahldraht ausgebildet, welcher einen kreisförmigen oder im Wesentlichen kreisförmigen Querschnitt aufweist.

Die verbesserten Clips 10 sind durch Umformen des Rohlings, insbesondere durch Pressformen, ausgebildet. Eine Querschnittsfläche der verbesserten Clips 10 im Bereich der Schraubenfeder 22 ist kleiner als im Bereich der Klemmarme 12 und 16.

Ferner ist eine Klemmarmlänge 88 der Klemmarme 12 und 16 größer als ein Schraubenfederdurchmesser 90 der Schraubenfeder 22.

### Bezugszeichenliste

- 10: Clip
- 12: erster Klemmarm
- 14: erste Klemmfläche
- 16: zweiter Klemmarm
- 18: zweite Klemmfläche
- 20: vorspannendes Element
- 22: Schraubenfeder
- 24: Schraubenfederlängsachse
- 26: erstes Schraubenfederende
- 28: zweites Schraubenfederende
- 30: Windung
- 32: erster Verbindungsabschnitt
- 34: zweiter Verbindungsabschnitt
- 36: Winkel
- 38: Klemmebene
- 40: erste Abkröpfung
- 42: zweite Abkröpfung
- 44: Schraubenfederebene
- 46: Dicke
- 48: Abschnitt
- 50: Abschnitt
- 52: erster Knickwinkel
- 54: Abschnitt
- 56: Abschnitt
- 58: zweiter Knickwinkel
- 60: dritter Knickwinkel
- 62: vierter Knickwinkel
- 64: Abflachung
- 66: Abflachung
- 68: Abflachungsebene
- 70: erster Windungsabschnitt
- 72: zweiter Windungsabschnitt
- 74: erste Windungsabschnittebene
- 76: zweite Windungsabschnittebene
- 78: Kröpfungsabschnitt
- 80: Kröpfungsabschnittswinkel
- 82: Windungsabschnittsspalt
- 84: erstes freies Ende
- 86: zweites freies Ende
- 88: Klemmarmlänge
- 90: Schraubenfederdurchmesser
- 92: Kreuzungsbereich
- 94: erstes Kröpfungsabschnittsende
- 96: zweites Kröpfungsabschnittsende

## Patentansprüche

1. Chirurgischer Clip (10) umfassend einen ersten Klemmarm (12), einen zweiten Klemmarm (16) und ein vorspannendes Element (20), welcher erste Klemmarm (12) eine erste Klemmfläche (14)definiert, welcher zweite Klemmarm (16) eine zweite Klemmfläche (18) definiert und welches vorspannende Element (20) die erste Klemmfläche (14) und die zweite Klemmfläche (18) in einer Grundstellung gegeneinander hält, insbesondere unter Vorspannung, wobei der erste und der zweite Klemmarm (12, 16) gegeneinander entgegen der Wirkung des vorspannenden Elements (20) verschwenkbar sind, wobei das vorspannende Element (20) in Form einer Schraubenfeder (22) ausgebildet ist, welche eine Schraubenfederlängsachse (24) definiert und ein erstes Schraubenfederende (26) und ein zweites Schraubenfederende (28) aufweist, wobei die Schraubenfeder (22) zwischen dem ersten Schraubenfederende (26) und dem zweiten Schraubenfederende (28) mindestens eine sich über einen Umfangswinkel von mehr als 360° erstreckende Windung (30) umfasst, wobei das erste Schraubenfederende (26) über einen ersten Verbindungsabschnitt (32) mit dem ersten Klemmarm (12) verbunden ist, wobei das zweite Schraubenfederende (28) über einen zweiten Verbindungsabschnitt (34) mit dem zweiten Klemmarm (16) verbunden ist, wobei die in der Grundstellung gegeneinander gehaltenen Klemmflächen (14, 18) eine Klemmebene (38) definieren und wobei die Schraubenfederlängsachse (24) parallel zur Klemmebene (38) verläuft, **dadurch gekennzeichnet, dass** der erste Verbindungsabschnitt (32) im Übergangsbereich zum ersten Schraubenfederende (26) abgekröpft ist unter Ausbildung einer Abkröpfung (40) und dass der zweite Verbindungsabschnitt (34) im Übergangsbereich zum zweiten Schraubenfederende (28) abgekröpft ist unter Ausbildung einer Abkröpfung (42), um die Schraubenfederlängsachse parallel zur Klemmfläche auszurichten, und dass die beiden Abkröpfungen (40, 42) als doppelte Abwinklungen des jeweiligen Verbindungsabschnitts (32, 34) zum jeweiligen Schraubenfederende (26, 28) ausgebildet sind.

2. Chirurgischer Clip nach Anspruch 1, **dadurch gekennzeichnet, dass** der erste Klemmarm (12) ein erstes freies Ende (84) aufweist, welches in einer Richtung vom vorspannenden Element (20) weg oder im Wesentlichen vom vorspannenden Element (20) weg weist.

3. Chirurgischer Clip nach einem der voranstehenden Ansprüche, **dadurch gekennzeichnet, dass**
a) der zweite Klemmarm (16) ein zweites freies Ende (86) aufweist, welches in einer Richtung vom vorspannenden Element (20) weg oder im Wesentlichen vom vorspannenden Element (20) weg weist, und/oder
b) der Clip (10) einen Kreuzungsbereich (92) umfasst und dass sich der erste Verbindungsabschnitt (32) und der zweite Verbindungsabschnitt (34) im Kreuzungsbereich (92) kreuzen.

4. Chirurgischer Clip nach einem der voranstehenden Ansprüche, **dadurch gekennzeichnet, dass** der erste Verbindungsabschnitt (32) und/oder der zweite Verbindungsabschnitt (34) eine auf den anderen Verbindungsabschnitt (32, 34) hin weisende Abflachung (64, 66) aufweisen, die insbesondere aneinander anliegen oder durch einen schmalen Spalt voneinander beabstandet sind.

5. Chirurgischer Clip nach Anspruch 4, **dadurch gekennzeichnet, dass**
a) die Abflachung (64, 66) des ersten und/oder zweiten Verbindungsabschnitts (32, 34) eine Abflachungsebene (68) definiert und dass die Abflachungsebene (68) parallel oder im Wesentlichen parallel zur Klemmebene (38) verläuft
und/oder
b) sich die Abflachung (64) des ersten Verbindungsabschnitts (32) direkt an den ersten Klemmarm (12) anschließt und/oder dass sich die Abflachung (66) des zweiten Verbindungsabschnitts (34) direkt an den zweiten Klemmarm (16) anschließt.

6. Chirurgischer Clip nach einem der voranstehenden Ansprüche, **dadurch gekennzeichnet, dass** der erste Verbindungsabschnitt (32) zwischen dem ersten Klemmarm (12) und dem ersten Schraubenfederende (26) um einen ersten Knickwinkel (52) abgewinkelt ist und/oder dass der zweite Verbindungsabschnitt (34) zwischen dem zweiten Klemmarm (16) und dem zweiten Schraubenfederende (28) um einen zweiten Knickwinkel (58) abgewinkelt ist.

7. Chirurgischer Clip nach Anspruch 6, **dadurch gekennzeichnet, dass**
a) der erste Knickwinkel (52) dem zweiten Knickwinkel (58) entspricht oder im Wesentlichen entspricht
und/oder
b) der erste und/oder der zweite Knickwinkel (52, 58) einen Innenwinkel in einem Bereich von etwa 90° bis etwa 120° definieren.

8. Chirurgischer Clip nach einem der voranstehenden Ansprüche, **dadurch gekennzeichnet, dass** der erste Verbindungsabschnitt (32) im Übergangsbereich zum ersten Klemmarm (12) um einen dritten Knickwinkel (60) abgewinkelt ist und/oder dass der zweite Verbindungsabschnitt (34) im Übergangsbereich zum zweiten Klemmarm (16) um einen vierten Knickwinkel (62) abgewinkelt ist.

9. Chirurgischer Clip nach Anspruch 8, **dadurch gekennzeichnet, dass**
a) der dritte Knickwinkel (60) dem vierten Knickwinkel (62) entspricht oder im Wesentlichen entspricht, insbesondere dem ersten und/oder zweiten Knickwinkel (52, 58),
und/oder
b) der dritte und/oder der vierte Knickwinkel (60, 62) einen Innenwinkel in einem Bereich von etwa 90° bis etwa 120° definieren.

10. Chirurgischer Clip nach einem der voranstehenden Ansprüche, **dadurch gekennzeichnet, dass** die mindestens eine Windung (30) mindestens einen ersten Windungsabschnitt (70) und mindestens einen zweiten Windungsabschnitt (72) umfasst und dass der mindestens eine erste Windungsabschnitt (70) eine erste Windungsabschnittsebene (74) definiert und/oder dass der mindestens eine zweite Windungsabschnitt (72) eine zweite Windungsabschnittsebene (76) definiert und dass der mindestens eine erste Windungsabschnitt (70) und der mindestens eine zweite Windungsabschnitt (72) über einen vom ersten Schraubenfederende (26) und vom zweiten Schraubenfederende (28) entfernten Kröpfungsabschnitt (78) miteinander verbunden sind.

11. Chirurgischer Clip nach Anspruch 10, **dadurch gekennzeichnet, dass**
a) der Kröpfungsabschnitt (78) geradlinig oder im Wesentlichen geradlinig ausgebildet ist und an einem ersten Kröpfungsabschnittsende (94) abgewinkelt mit dem mindestens einen ersten Windungsabschnitt (70) verbunden ist und an einem zweiten Kröpfungsabschnittsende (96) abgewinkelt mit dem mindestens einen zweiten Windungsabschnitt (72) verbunden ist
und/oder
b) der Kröpfungsabschnitt (78) quer, insbesondere unter einem Kröpfungsabschnittswinkel (80) von etwa 45°, zur ersten und/oder zweiten Windungsabschnittsebene (74, 76) verläuft
und/oder
c) die erste Windungsabschnittsebene (74) und/oder die zweite Windungsabschnittsebene (76) senkrecht zur Schraubenfederlängsachse (24) verlaufen
und/oder
d) sich der mindestens eine erste Windungsabschnitt (70) über einen ersten Windungsabschnittsumfangswinkel von weniger als 360°, insbesondere weniger als 300°, erstreckt und/oder dass sich der mindestens eine zweite Windungsabschnitt (72) über einen zweiten Windungsabschnittsumfangswinkel von weniger als 360°, insbesondere weniger als 300°, erstreckt
und/oder
e) der mindestens eine erste Windungsabschnitt (70) und der mindestens eine zweite Windungsabschnitt (72) durch einen Windungsabschnittsspalt (82) voneinander getrennt sind.

12. Chirurgischer Clip nach einem der voranstehenden Ansprüche, **dadurch gekennzeichnet, dass** die Schraubenfeder (22) einen Schraubenfederdurchmesser (90) definiert, dass der erste Klemmarm (12) und der zweite Klemmarm (16) eine Klemmarmlänge (88) aufweisen und dass der Schraubenfederdurchmesser (90) kleiner als die Klemmarmlänge (88) ist.

13. Chirurgischer Clip nach einem der voranstehenden Ansprüche, **dadurch gekennzeichnet, dass** die erste Klemmfläche (14) und/oder die zweite Klemmfläche (18) eine Klemmflächenstruktur aufweisen, insbesondere eine makroskopische und/oder eine mikroskopische,
wobei weiter insbesondere die Klemmflächenstruktur Klemmvorsprünge und/oder Klemmausnehmungen umfasst, insbesondere linien- und/oder punktförmige.

14. Chirurgischer Clip nach einem der voranstehenden Ansprüche, **dadurch gekennzeichnet, dass** der Clip (10)
a) einstückig, insbesondere monolithisch, ausgebildet ist
und/oder
b) aus einem Federstahldraht, insbesondere mit kreisförmigem oder im Wesentlichen kreisförmigem Querschnitt, durch Umformen, insbesondere durch Pressformen, ausgebildet ist.

15. Chirurgischer Clip nach einem der voranstehenden Ansprüche, **dadurch gekennzeichnet, dass** eine Querschnittsfläche des Clips (10) im Bereich der Schraubenfeder (22) kleiner ist als im Bereich des ersten und/oder zweiten Verbindungsabschnitts (32, 34) und/oder im Bereich des ersten und/oder des zweiten Klemmarms (12, 16).

## Claims

1. Surgical clip (10), comprising a first clamping arm (12), a second clamping arm (16), and a biasing element (20), which first clamping arm (12) defines a first clamping surface (14), which second clamping arm (16) defines a second clamping surface (18), and which biasing element (20) holds the first clamping surface (14) and the second clamping surface (18) against each other in a basic position, in particular under bias, wherein the first and the second clamping arm (12, 16) are pivotable against each other counter to the action of the biasing element (20), wherein the biasing element (20) is configured in the form of a coil spring (22) which defines a coil spring longitudinal axis (24) and has a first coil spring end (26) and a second coil spring end (28), wherein the coil spring (22) comprises between the first coil spring end (26) and the second coil spring end (28) at least one winding (30) extending over a circumferential angle of more than 360°, wherein the first coil spring end (26) is connected to the first clamping arm (12) by way of a first connecting portion (32), wherein the second coil spring end (28) is connected to the second clamping arm (16) by way of a second connecting portion (34), wherein the clamping surfaces (14, 18) held against each other in the basic position define a clamping plane (38), and wherein the coil spring longitudinal axis (24) runs parallel to the clamping plane (38), **characterized in that** the first connecting portion (32) is cranked in the transition region to the first coil spring end (26) thereby forming a crank (40), and **in that** the second connecting portion (34) is cranked in the transition region to the second coil spring end (28) thereby forming a crank (42) in order to orient the coil spring longitudinal axis in parallel to the clamping surface, and **in that** the two cranks (40, 42) are formed as double anglings of the respective connecting portions (32, 34) in relation to the respective coil spring end (26, 28).

2. Surgical clip in accordance with Claim 1, **characterized in that** the first clamping arm (12) has a first free end (84) which points in a direction away from the biasing element (20) or substantially away from the biasing element (20).

3. Surgical clip in accordance with any one of the preceding Claims, **characterized in that**
a) the second clamping arm (16) has a second free end (86) which points in a direction away from the biasing element (20) or substantially away from the biasing element (20)
and/or
b) the clip (10) comprises an intersection region (92), and **in that** the first connecting portion (32) and the second connecting portion (34) intersect in the intersection region (92).

4. Surgical clip in accordance with any one of the preceding Claims, **characterized in that** the first connecting portion (32) and/or the second connecting portion (34) have a flattened portion (64, 66) facing toward the other connecting portion (32, 34), which in particular abut against each other or are spaced apart from each other by a narrow gap.

5. Surgical clip in accordance with Claim 4, **characterized in that**
a) the flattened portion (64, 66) of the first and/or second connecting portion (32, 34) defines a flattened portion plane (68), and **in that** the flattened portion plane (68) runs parallel or substantially parallel to the clamping plane (38)
and/or
b) the flattened portion (64) of the first connecting portion (32) directly adjoins the first clamping arm (12) and/or **in that** the flattened portion (66) of the second connecting portion (34) directly adjoins the second clamping arm (16).

6. Surgical clip in accordance with any one of the preceding Claims, **characterized in that** the first connecting portion (32) is angled by a first bend angle (52) between the first clamping arm (12) and the first coil spring end (26), and/or **in that** the second connecting portion (34) is angled by a second bend angle (58) between the second clamping arm (16) and the second coil spring end (28).

7. Surgical clip in accordance with Claim 6, **characterized in that**
a) the first bend angle (52) corresponds to or substantially corresponds to the second bend angle (58)
and/or
b) the first and/or the second bend angle (52, 58) define an interior angle in a range of about 90° to about 120°.

8. Surgical clip in accordance with any one of the preceding Claims, **characterized in that** the first connecting portion (32) is angled by a third bend angle (60) in the transition region to the first clamping arm (12), and/or **in that** the second connecting portion (34) is angled by a fourth bend angle (62) in the transition region to the second clamping arm (16).

9. Surgical clip in accordance with Claim 8, **characterized in that**
a) the third bend angle (60) corresponds to or substantially corresponds to the fourth bend angle (62), in particular to the first and/or second bend angle (52, 58),
and/or
b) the third and/or the fourth bend angle (60, 62) define an interior angle in a range of about 90° to about 120°.

10. Surgical clip in accordance with any one of the preceding Claims, **characterized in that** the at least one winding (30) comprises at least one first winding portion (70) and at least one second winding portion (72), and **in that** the at least one first winding portion (70) defines a first winding portion plane (74), and/or **in that** the at least one second winding portion (72) defines a second winding portion plane (76), and **in that** the at least one first winding portion (70) and the at least one second winding portion (72) are connected to each other by way of a cranked portion (78) remote from the first coil spring end (26) and from the second coil spring end (28).

11. Surgical clip in accordance with Claim 10, **characterized in that**
a) the cranked portion (78) is of rectilinear or substantially rectilinear configuration and is connected to the at least one first winding portion (70) at an angle at a first cranked portion end (94) and is connected to the at least one second winding portion (72) at an angle at a second cranked portion end (96)
and/or
b) the cranked portion (78) extends transversely, in particular at a cranked portion angle (80) of about 45°, to the first and/or second winding portion plane (74, 76)
and/or
c) the first winding portion plane (74) and/or the second winding portion plane (76) extend perpendicularly to the coil spring longitudinal axis (24)
and/or
d) the at least one first winding portion (70) extends over a first winding portion circumferential angle of less than 360°, in particular less than 300°, and/or **in that** the at least one second winding portion (72) extends over a second winding portion circumferential angle of less than 360°, in particular less than 300°,
and/or
e) the at least one first winding portion (70) and the at least one second winding portion (72) are separated from each other by a winding portion gap (82).

12. Surgical clip in accordance with any one of the preceding Claims, **characterized in that** the coil spring (22) defines a coil spring diameter (90), **in that** the first clamping arm (12) and the second clamping arm (16) have a clamping arm length (88), and **in that** the coil spring diameter (90) is less than the clamping arm length (88).

13. Surgical clip in accordance with any one of the preceding Claims, **characterized in that** the first clamping surface (14) and/or the second clamping surface (18) have a clamping surface structure, in particular a macroscopic and/or a microscopic clamping surface structure, wherein in particular the camping surface structure comprises clamping projections and/or clamping recesses, in particular linear and/or pointshaped clamping projections and/or clamping recesses.

14. Surgical clip in accordance with any one of the preceding Claims, **characterized in that** the clip (10)
a) is formed as one piece, in particular monolithically,
and/or
b) is made from a spring steel wire, in particular with a circular or substantially circular cross section, by means of forming, in particular by means of press forming.

15. Surgical clip in accordance with any one of the preceding Claims, **characterized in that** a cross sectional area of the clip (10) in the region of the coil spring (22) is smaller than in the region of the first and/or second connecting portion (32, 34) and/or in the region of the first and/or the second clamping arm (12, 16).

## Revendications

1. Clip (10) chirurgical comprenant un premier bras de serrage (12), un second bras de serrage (16) et un élément de précontrainte (20), ledit premier bras de serrage (12) définit une première surface de serrage (14), ledit second bras de serrage (16) définit une seconde surface de serrage (18) et lequel élément de précontrainte (20) maintient la première surface de serrage (14) et la seconde surface de serrage (18) l'une contre l'autre dans une position de base, en particulier sous une précontrainte, les premier et second bras de serrage (12, 16) pouvant pivoter l'un par rapport à l'autre à l'encontre de l'action de l'élément de précontrainte (20), l'élément de précontrainte (20) se présentant sous la forme d'un ressort hélicoïdal (22), lequel définit un axe longitudinal de ressort hélicoïdal (24) et présente une première extrémité de ressort hélicoïdal (26) et une seconde extrémité de ressort hélicoïdal (28), le ressort hélicoïdal (22) comprenant entre la première extrémité de ressort hélicoïdal (26) et la seconde extrémité de ressort hélicoïdal (28) au moins une spire (30) s'étendant sur un angle périphérique supérieur à 360°, la première extrémité de ressort hélicoïdal (26) étant reliée au premier bras de serrage (12) par l'intermédiaire d'une première partie de liaison (32), la seconde extrémité de ressort hélicoïdal (28) étant reliée au second bras de serrage (16) par l'intermédiaire d'une seconde partie de liaison (34), les surfaces de serrage (14, 18) maintenues l'une contre l'autre dans la position de base définissant un plan de serrage (38) et l'axe longitudinal de ressort hélicoïdal (24) se déroule en parallèle du plan de serrage (38), **caractérisée en ce que** la première partie de liaison (32) est coudée dans la zone de transition vers la première extrémité de ressort hélicoïdal (26) formant ainsi un coude (40) et **en ce que** la seconde partie de liaison (34) est coudée dans la zone de transition vers la seconde extrémité de ressort hélicoïdal (28) formant ainsi un coude (42), pour orienter l'axe longitudinal de ressort hélicoïdal en parallèle de la surface de serrage, et **en ce que** les deux coudes (40, 42) sont conçus en tant que flexions doubles de la partie de liaison (32, 34) respective vers l'extrémité de ressort hélicoïdal (26, 28) respective.

2. Clip chirurgical selon la revendication 1, **caractérisée en ce que** le premier bras de serrage (12) présente une première extrémité libre (84), qui est orientée dans une direction s'éloignant de l'élément de précontrainte (20) ou s'éloignant essentiellement de l'élément de précontrainte (20).

3. Clip chirurgical selon l'une des revendications précédentes, **caractérisée en ce que**
a) le second bras de serrage (16) présente une seconde extrémité libre (86), qui est orientée dans une direction s'éloignant de l'élément de précontrainte (20) ou s'éloignant essentiellement de l'élément de précontrainte (20),
et/ou
b) le clip (10) comprend une zone de croisement (92) et **en ce que** la première partie de liaison (32) et la seconde partie de liaison (34) se croisent dans la zone de croisement (92).

4. Clip chirurgical selon l'une des revendications précédentes, **caractérisée en ce que** la première partie de liaison (32) et/ou la seconde partie de liaison (34) présentent un méplat (64, 66) orienté vers l'autre partie de liaison (32, 34), qui sont en particulier adjacentes l'une à l'autre ou espacées l'une de l'autre par une fente étroite.

5. Clip chirurgical selon la revendication 4, **caractérisée en ce que**
a) le méplat (64, 66) de la première et/ou seconde partie de liaison (32, 34) définit un plan de méplat (68) et **en ce que** le plan de méplat (68) s'étend en parallèle ou essentiellement en parallèle du plan de serrage (38)
et/ou
b) le méplat (64) de la première partie de liaison (32) se raccorde directement au premier bras de serrage (12) et/ou **en ce que** le méplat (66) de la seconde partie de liaison (34) se raccorde directement au second bras de serrage (16).

6. Clip chirurgical selon l'une des revendications précédentes, **caractérisée en ce que** la première partie de liaison (32) entre le premier bras de serrage (12) et la première extrémité de ressort hélicoïdal (26) est coudée selon un premier angle de pliage (52) et/ou **en ce que** la seconde partie de liaison (34) entre le second bras de serrage (16) et la seconde extrémité de ressort hélicoïdal (28) est coudée selon un deuxième angle de pliage (58).

7. Clip chirurgical selon la revendication 6, **caractérisée en ce que**
a) le premier angle de pliage (52) correspond ou correspond essentiellement au deuxième angle de pliage (58)
et/ou
b) le premier et/ou le deuxième angles de pliage (52, 58) définissent un angle intérieur dans une plage de 90° environ à 120° environ.

8. Clip chirurgical selon l'une des revendications précédentes, **caractérisée en ce que** la première partie de liaison (32) est coudée selon un troisième angle de pliage (60) dans la zone de transition vers le premier bras de serrage (12) et/ou **en ce que** la seconde partie de liaison (34) est coudée selon un quatrième angle de pliage (62) dans la zone de transition vers le second bras de serrage (16).

9. Clip chirurgical selon la revendication 8, **caractérisée en ce que**
a) le troisième angle de pliage (60) correspond ou correspond essentiellement au quatrième angle de pliage (62), en particulier au premier et/ou deuxième angle de pliage (52, 58),
et/ou
b) le troisième et/ou le quatrième angles de pliage (60, 62) définissent un angle intérieur dans une plage de 90° environ à 120° environ.

10. Clip chirurgical selon l'une des revendications précédentes, **caractérisée en ce que** la au moins une spire (30) comprend au moins une première partie de spire (70) et au moins une seconde partie de spire (72) et **en ce que** la au moins une première partie de spire (70) définit un premier plan de partie de spire (74) et/ou **en ce que** la au moins une seconde partie de spire (72) définit un second plan de partie de spire (76) et **en ce que** la au moins une première partie de spire (70) et la au moins une seconde partie de spire (72) sont reliées l'une à l'autre par l'intermédiaire d'une partie coudée (78) éloignée de la première extrémité de ressort hélicoïdal (26) et de la second extrémité de ressort hélicoïdal (28).

11. Clip chirurgical selon la revendication 10, **caractérisée en ce que**
a) la partie coudée (78) est formée rectiligne ou essentiellement rectiligne et est reliée à une première extrémité de partie coudée (94) de manière coudée à ladite au moins une première partie de spire (70) et est reliée à une seconde extrémité de partie coudée (96) de manière coudée à ladite au moins une seconde partie de spire (72)
et/ou
b) la partie coudée (78) s'étend transversalement, en particulier sous un angle de partie coudée (80) d'environ 45°, par rapport au premier et/ou second plan de partie de spire (74, 76)
et/ou
c) le premier plan de partie de spire (74) et/ou le second plan de partie de spire (76) s'étendent perpendiculairement à l'axe longitudinal de ressort hélicoïdal (24)
et/ou
d) la au moins une première partie de spire (70) s'étend sur un premier angle périphérique de partie de spire inférieur à 360°, en particulier inférieur à 300°, et/ou **en ce que** la au moins une seconde partie de spire (72) s'étend sur un deuxième angle périphérique de partie de spire inférieur à 360°, en particulier inférieur à 300°,
et/ou
e) la au moins une première partie de spire (70) et la au moins une seconde partie de spire (72) sont séparées l'une de l'autre par une fente de partie de spire (82).

12. Clip chirurgical selon l'une des revendications précédentes, **caractérisée en ce que** le ressort hélicoïdal (22) définit un diamètre de ressort hélicoïdal (90), **en ce que** le premier bras de serrage (12) et le second bras de serrage (16) présentent une longueur de bras de serrage (88) et **en ce que** le diamètre de ressort hélicoïdal (90) est inférieur à la longueur de bras de serrage (88).

13. Clip chirurgical selon l'une des revendications précédentes, **caractérisée en ce que** la première surface de serrage (14) et/ou la seconde surface de serrage (18) présentent une structure de surface de serrage, en particulier macroscopique et/ou microscopique,
dans lequel davantage en particulier la structure de surface de serrage comprend des saillies de serrage et/ou des évidements de serrage, en particulier en particulier linéaires et/ou ponctuels.

14. Clip chirurgical selon l'une des revendications précédentes, **caractérisée en ce que** le clip (10)
a) est conçue en une seule pièce, en particulier de façon monolithique,
et/ou
b) est réalisé par moulage, en particulier par moulage par compression, à partir d'un fil d'acier à ressort, en particulier de section circulaire ou essentiellement circulaire.

15. Clip chirurgical selon l'une des revendications précédentes, **caractérisée en ce qu'**une surface en coupe transversale du clip (10) dans la zone du ressort hélicoïdal (22) est inférieure à celle dans la zone de la première et/ou seconde partie de liaison (32, 34) et/ou à celle dans la zone du premier et/ou du second bras de serrage (12, 16).
